# EUROPÄISCHE PATENTANMELDUNG

(11) **EP 3 900 759 A1**
(43) Veröffentlichungstag der Anmeldung: **27.10.2021**
(21) Anmeldenummer: 20170759.3
(22) Anmeldetag: 22.04.2020
(51) Int. Cl.: A61M 5/20, A61M 5/24, A61M 5/315

(54) **AUTOINJEKTOR MIT AUSSCHÜTTFREIGABE**

(71) Anmelder: Ypsomed AG, 3401 Burgdorf (CH)
(72) Erfinder: Tschirren, Markus, 3400 Burgdorf (CH); Kalbermatter, Gabriel, 3400 Burgdorf (CH); Urbanek, Leos, 3012 Bern (CH); Allenspach, Marcel, 3400 Burgdorf (CH)
(74) Vertreter: Meier Obertüfer, Jürg

(57) **Zusammenfassung**

Die Erfindung betrifft einen Autoinjektor mit einem Gehäuse, einem im Gehäuse axial fixierten Produktbehälter (11), einer Torsionsfeder (20a), einem Antriebselement (21, 21a, 21b), einem Vortriebselement (22, 22a, 22b), und einer Nadelschutzhülse (14), welche bei Anpressen des Autoinjektors an eine Injektionsstelle die Nadelschutzhülse (14) eine Betätigungsbewegung in proximale Richtung ausführt. Zur Ausschüttung von Flüssigkeit aus dem Produktbehälter (11) durch eine Injektionsnadel (11b) in die Injektionsstelle versetzt die Torsionsfeder (20a) das Antriebselement (21) in Rotation und das rotierende Antriebselement bewirkt eine Bewegung des Vortriebselements (22) und eines Kolbens im Produktbehälter in distaler Richtung. Der Autoinjektor umfasst eine Kupplung, welche als Folge der Betätigungsbewegung der Nadelschutzhülse (14) das Antriebselement (21) zur Rotation freigibt. Die Kupplung umfasst ein erstes Kupplungselement (23c), welches über eine axiale Sperrfläche (24a, 24b) in ein zweites Kupplungselement (21c) eingreift, wobei dieser Eingriff durch einen axialen Kupplungshub der beiden Kupplungselemente lösbar ist.

## Beschreibung

### TECHNISCHES GEBIET

Die vorliegende Erfindung betrifft das Gebiet der medizinischen Injektionsgeräte zur Verabreichung von flüssigen Substanzen, insbesondere von Medikamenten oder medizinischen Substanzen wie Insulin- und Hormonpräparationen. Die Erfindung bezieht sich auf einen Autoinjektor mit einem Energiespeicher zur Ausschüttung einer vorgegebenen Dosis aus einem einmalig genutzten Produktbehälter.

### HINTERGRUND DER ERFINDUNG

Injektionsgeräte oder Injektionsvorrichtungen zum vereinfachten Verabreichen einer Substanz umfassen unter anderem so genannte Autoinjektoren, welche einen Energiespeicher aufweisen, mit welchem die Ausschüttung automatisch, das heisst ohne extern von einem Anwender zuzuführende oder aufzuwendende Kraft, durchgeführt werden kann. Der Energiespeicher speichert die für eine automatische Substanzabgabe erforderliche Energie vorteilhaft in mechanischer Form. Ein solcher Energiespeicher kann eine Feder sein, welche in einem gespannten Zustand in das Injektionsgerät eingebaut wird und durch Entspannen Energie abgibt. Die Energieabgabe erfolgt an eine Kolbenstange oder ein Druckelement, welches einen Kolben in einen Produktbehälter einschiebt. Der Energiespeicher kann auch vorgesehen sein um den Vorgang des Einstechens einer Injektionsnadel zu automatisieren. Alternativ kann der Einstechvorgang manuell erfolgen, also ausschliesslich durch einen Anwender, ohne hierfür in dem Injektionsgerät gespeicherte Energie zu verwenden.

Das Injektionsgerät kann einen Produktbehälterhalter zur Aufnahme eines Produktbehälters umfassen, wobei in dem Produktbehälterhalter der Produktbehälter radial, axial und vorzugsweise auch drehfest gehalten werden kann. Der Produktbehälterhalter kann mit dem Gehäuse der Injektionsvorrichtung axial- und drehfest verbunden sein, oder bei einem Einstech- und/oder Nadelrückzugsvorgang relativ zum Gehäuse bewegbar sein. Der Produktbehälter kann eine Karpule zur einmaligen oder wiederholt lösbaren Verbindung mit Einweg-Injektionsnadeln oder eine Einweg-Fertigspritze mit einer damit unlösbar verbundenen Injektionsnadel sein. Der Produktbehälter weist einen hohlzylindrischen Produktbehälterabschnitt auf, der einen Kolben oder Stopfen verschiebbar lagert. Der Kolben kann mit dem Innenumfang des Produktbehälterabschnitts einen Dichtspalt bilden und mittels Kolbenstange in eine distale Richtung verschoben werden, um über die Injektionsnadel Produkt aus dem Produktbehälter abzugeben.

Das Injektionsgerät kann eine Nadelschutzhülse aufweisen, die nach erfolgter Injektion distal über das distale Ende der Injektionsnadel steht oder relativ zu dem Gehäuse unter Entspannung einer Nadelschutzhülsenfeder in diese Position verschoben wird, um den versehentlichen Zugriff auf die Injektionsnadel zu verhindern und dadurch ein Verletzungsrisiko zu verringern. Bei einem Autoinjektor kann die Nadelschutzhülse auch als Auslöseelement zum Auslösen der Produktausschüttung dienen, wobei die Nadelschutzhülse hierzu relativ zu dem Gehäuse in die proximale Richtung verschoben wird. Alternativ kann die Auslösung des Autoinjektors durch Betätigen eines Auslöseknopfs des Autoinjektors erreicht werden, wobei die Nadelschutzhülse vor dem Gebrauch des Autoinjektors zumindest als Sichtschutz dient.

Die Patentanmeldung WO 2016/205963 beschreibt einen beispielhaften Autoinjektor, umfassend ein Gehäuse mit Längsachse und einen axial fest im Gehäuse angeordneten Produktbehälter. Der Autoinjektor umfasst weiter eine in einer Längsrichtung zwischen einer proximalen und einer distalen Position verschiebbare Nadelschutzhülse, welche mit einer Nadelschutzfeder gekoppelt ist. Eine Spiral- oder Triebfeder, in welcher Energie für das automatische Ausschütten von Produkt gespeichert werden kann, ist über ein erstes Ende mit dem Gehäuse und über ein zweites Ende rotationsfest mit einem koaxial zur Längsachse angeordneten Antriebselement in Form einer rotierenden Gewindestange verbunden. Die Gewindestange greift über ein Gewinde in ein im Gehäuse nicht rotierendes Vortriebsglied in Form einer Vortriebshülse, welche bei einer Verschiebung in distale Richtung den Stopfen des Produktbehälters mit einer zumindest annähernd konstanten Ausschüttgeschwindigkeit mitbewegt. Vor der Ausschüttung greift ein Halteelement mit zwei flexiblen Haltearmen in Ausnehmungen des Vortriebsglieds ein und verhindert eine Bewegung desselben. Die Arme des Halteelements werden dabei durch eine Sperrhülse im besagten Eingriff gehalten. Beim Auslösen der Ausschüttung wird die Sperrhülse durch eine proximale Bewegung der Nadelschutzhülse von der Position der Ausnehmungen wegbewegt, so dass sich die Haltearme radial lösen können und das Vortriebsglied freigeben. Vor der Ausschüttung und unter Umständen während mehrerer Jahre liegt das Drehmoment der Spiralfeder über das Antriebselement am proximalen Ende des Vortriebsglieds und die Vortriebskraft am Halteelement an, was zu einer statischen Materialbelastung des Vortriebsglieds und der Haltearme führt und mögliche Ermüdungserscheinungen zur Folge haben kann.

Die Patentanmeldung WO 2009/037141 A1 beschreibt einen Autoinjektor mit Spritzenbewegung für hochviskose Medikamente, bei welchem eine Nadelschutzhülse bei Aufsetzen auf die Injektionsstelle eine Auslösehülse um einen geringen Auslösehub in proximale Richtung schiebt und dabei eine radiale Verzahnung zwischen der Auslösehülse und einem Antriebsglied gelöst wird. Das Antriebsglied wird durch eine Spiralfeder angetrieben und schiebt über eine Gewindeverbindung mit einem proximalen Gewinde einer innenliegenden Kolbenstange diese in axialer Richtung, was zuerst für eine automatische Einstechbewegung und anschliessend für eine Medikamentenausschüttung sorgt. Die Kolbenstange umfasst weiter einen distalen Teil mit einem grösseren Durchmesser als der gewindetragende proximale Teil der Kolbenstange.

Der Begriff "Produkt", "Medikament" oder "medizinische Substanz" umfasst im vorliegenden Zusammenhang jede fliessfähige medizinische Formulierung, welche geeignet ist zur kontrollierten Verabreichung mittels einer Kanüle oder Hohlnadel in subkutanes oder intramuskuläres Gewebe, beispielsweise eine Flüssigkeit, eine Lösung, ein Gel oder eine feine Suspension enthaltend einen oder mehrere medizinische Wirkstoffe. Ein Medikament kann also eine Zusammensetzung mit einem einzigen Wirkstoff oder eine vorgemischte oder co-formulierte Zusammensetzung mit mehreren Wirkstoffen aus einem einzelnen Behälter sein. Der Begriff umfasst insbesondere Arzneien wie Peptide (z.B. Insuline, Insulin enthaltende Medikamente, GLP-1 enthaltende sowie abgeleitete oder analoge Zubereitungen), Proteine und Hormone, biologisch gewonnene oder aktive Wirkstoffe, Wirkstoffe auf Basis von Hormonen oder Genen, Nährformulierungen, Enzyme und weitere Substanzen sowohl in fester (suspendierter) oder flüssiger Form. Der Begriff umfasst weiter auch Polysaccharide, Vakzine, DNS oder RNS oder Oligonukleotide, Antikörper oder Teile von Antikörpern sowie geeignete Basis-, Hilfs- und Trägerstoffe.

Der Begriff "distal" bezeichnet eine zum vorderen, einstechseitigen Ende der Verabreichungsvorrichtung beziehungsweise zur Spitze der Injektionsnadel hin gerichtete Seite oder Richtung. Demgegenüber bezeichnet die Angabe "proximal" eine zum hinteren, dem einstechseitigen Ende gegenüberliegenden Ende der Verabreichungsvorrichtung hin gerichtete Seite oder Richtung.

Unter den Begriffen "Injektionssystem" oder "Injektor" wird in der vorliegenden Beschreibung eine Vorrichtung verstanden, bei der die Injektionsnadel nach erfolgter Abgabe einer kontrollierten Menge der medizinischen Substanz aus dem Gewebe entfernt wird. Somit verbleibt bei einem Injektionssystem oder bei einem Injektor im Unterschied zu einem Infusionssystem die Injektionsnadel nicht über einen längeren Zeitraum von mehreren Stunden im Gewebe.

### DARSTELLUNG DER ERFINDUNG

Es ist Aufgabe der Erfindung einen Autoinjektor mit einem Torsionsfederantrieb zum automatischen Ausschütten einer Flüssigkeit aus einer gehäusefesten Fertigspritze anzugeben, bei welchem ein Drehmoment der Torsionsfeder zuverlässig bis zur geplanten Ausschüttung blockiert oder absorbiert wird. Die Aufgabe wird gelöst durch einen Autoinjektor und eine Antriebseinheit mit den Merkmalen der unabhängigen Ansprüche. Bevorzugte Ausführungsformen der Erfindung sind Gegenstand der abhängigen Ansprüche.

Ein erfindungsgemässer Autoinjektor umfasst ein einteiliges oder mehrteiliges Gehäuse mit einer Längsachse und eine vorgefüllte Fertigspritze mit einem Produktbehälter und einer daran unlösbar befestigten Injektionsnadel oder Kanüle. Die Fertigspritze ist im Gehäuse axial nicht verschiebbar aufgenommen, wobei eine Spitze der Injektionsnadel um eine Einstechtiefe in distaler Richtung über ein distales Gehäuseende vorsteht. Der Autoinjektor umfasst weiter eine zur einmaligen Ausschüttung eines maximalen Inhalts des Produktbehälters vorgespannte Torsionsfeder, ein Antriebselement, ein Vortriebselement, und eine Nadelschutzhülse. Zur Ausschüttung von Flüssigkeit aus dem Produktbehälter durch die Injektionsnadel versetzt die Torsionsfeder das Antriebselement in Rotation um die Längsachse, und das rotierende Antriebselement bewirkt eine Linearbewegung des Vortriebselements zur Verschiebung eines Kolbens im Produktbehälter. Die Nadelschutzhülse wird beim Anpressen des Autoinjektors an eine Injektionsstelle und dem dadurch bewirkten Einstechen der Injektionsnadel in die Injektionsstelle um einen Betätigungshub in proximale Richtung bewegt und startet oder ermöglicht dadurch eine Ausschüttung von Flüssigkeit. Der Betätigungshub der Nadelschutzhülse entspricht dabei mindestens der Einstechtiefe der Injektionsnadel.

Der Autoinjektor umfasst schliesslich eine Kupplung mit vom Vortriebselement unabhängigen Kupplungselementen, welche vor der Ausschüttung eine Drehung des Antriebselementes blockiert und als Folge der Bewegung der Nadelschutzhülse beim Einstechen das Antriebselement oder ein damit drehfest verbundenes Rotationselement zur Rotation freigibt. Die Kupplung blockiert also nicht die Linearbewegung des Vortriebselements, sondern unmittelbar das Drehmoment der Torsionsfeder am Antriebselement. Dadurch wird vermieden, dass die drehfeste Verbindung oder Kopplung von Antriebs- und Vortriebselement während der gesamten Lagerdauer des Autoinjektors belastet wird und dadurch eventuell Schaden nimmt. Weiter ist im Vortriebselement keine Ausnehmung vorgesehen für den Eingriff eines Halteelements, das Vortriebselement kann dadurch bei der Montage auf ein Gewinde entsprechend einem vorgesehenen Ausschütthub beziehungsweise einer axialen Ausgangsposition des Stopfens unterschiedlich weit geschraubt werden. Für teilgefüllte Fertigspritzen wird das Vortriebsglied nicht über die gesamte Länge des Getriebes geschraubt, dadurch sind Bruchteile eines maximalen Ausschütthubs einfach einstellbar.

In einer bevorzugten Ausführungsform umfasst die Kupplung ein erstes, nach innen gerichtetes Kupplungselement, welches über gegen Rotation um die Längsachse wirkende und bevorzugt parallel zur Längsachse ausgerichtete Sperrflächen in ein zweites, nach aussen gerichtetes Kupplungselement selektiv eingreift, wobei dieser Eingriff durch einen axialen Kupplungshub oder eine axiale Relativbewegung der beiden Kupplungselemente gelöst wird. Durch diese axiale Bewegung wird radial kein zusätzlicher Platz benötigt im Autoinjektor, im Gegensatz zu einer radialen Entkopplung bei welcher ein erstes Kupplungselement in radialer Richtung von einem zweiten Kupplungselement wegbewegt wird.

Der Kupplungshub kann manuell oder federunterstützt erfolgen, ohne Übersetzung entspricht der Kupplungshub einem Betätigungshub der Nadelschutzhülse beim Einstechen, und somit mindestens einer Einstechtiefe der Injektionsnadel, welche ihrerseits mindestens 3 mm und bevorzugt mindestens 5 mm beträgt. Das Drehmoment der Torsionsfeder wird über die Kupplungselemente mit Sperrflächen parallel zur Längsachse des Autoinjektors gesperrt oder aufgenommen. Eine axiale Ausdehnung dieser Sperrflächen unterschreitet ein Ausmass des Kupplungshubs, und beträgt jedoch vorteilhafterweise mindestens 2 mm, bevorzugt mindestens 4 mm, und kann tatsächlich ebenfalls mindestens der Einstechtiefe entsprechen.

In einer bevorzugten Ausführungsform weist das Vortriebselement einen nichtrotationssymmetrischen Querschnitt auf mit einem Axialführungselement in Form einer Nut, Ebene, oder Rippe parallel zur Längsachse, über welche das Vortriebselement durch das Gehäuse oder ein im Gehäuse rotationsfest aufgenommenes Führungsgegenelement axial linear geführt ist. Das Vortriebselement ist dabei über ein Gewinde an das Antriebselement gekoppelt und bewegt sich ausschliesslich axial in Ausschüttrichtung und nicht etwa in einer schraubenden Vortriebsbewegung. Dadurch wird Reibung zwischen einer distalen Stirnfläche des Vortriebsglieds und dem Kolben vermieden, zudem ist es einfacher, das Antriebselement und/oder das Vortriebselement mit einem zur Erzeugung einer geänderten Ausschüttkraft angepassten Gewinde oder mit einer speziellen Gewindeflächenbeschichtung zu versehen als das Gehäuse. Das Vortriebselement kann beim Einschrauben in oder beim Aufschrauben auf das Gewinde des Antriebselements entsprechend einem vorgesehenen Ausschütthub beziehungsweise einer axialen Ausgangsposition des Stopfens unterschiedlich weit geschraubt werden. Falls das Gehäuse und/oder das Axialführungselement mehrere diskrete Rotationspositionen des Vortriebselements erlaubt, wenn also das Vortriebselement in einer ersten wie auch in einer zweiten, gegenüber der ersten um 180° oder gar nur um 90° um die Längsachse gedrehten Orientierung durch das Gehäuse geführt werden kann, ist eine Teil- Ausschütthub mit einer Auflösung von weniger als einem Gewindegang, speziell von einem halben oder gar nur einem Viertel eines Gewindegangs, einstellbar.

Weiter bevorzugt weist das Antriebselement eine Gewindestange mit einem Aussengewinde und das Vortriebselement eine Vortriebshülse mit dem Axialführungselement und einem an das Aussengewinde angepassten Innengewinde auf. Das Innengewinde erstreckt sich über eine dem maximalen Ausschütthub entsprechende Länge, und das Aussengewinde ist am proximalen Ende der Gewindestange auf wenige Windungen verkürzt oder umfasst ein Gewindesegment von weniger als einer Windung. Alternativ und in spritzgusstechnisch vorteilhafter Weise erstreckt sich das Aussengewinde über die maximale Länge des Ausschütthubs und ist das Innengewinde entsprechend verkürzt. Bei Rotation der Gewindestange wird die nichtrotierend gelagerte Vortriebshülse in distale Richtung geschoben, was einfacher zu bewerkstelligen ist als die Linearführung einer Gewindestange.

Die Torsionsfeder als elastisches Mittel zur Erzeugung eines Drehmoments ist bevorzugt eine Spiralfeder, kann aber auch als Blattfeder, Triebfeder, Konusfeder, Wendeltorsionsfeder, Drehstab oder Kombinationen davon ausgebildet sein. Sie ist bei Auslieferung beziehungsweise vor Inbetriebnahme des Autoinjektors maximal oder hinreichend vorgespannt für eine einmalige Ausschüttung des gesamten oder zumindest eines vorbestimmten Inhalts des Produktbehälters. Der Autoinjektor verfügt dementsprechend nicht über einen Dosiswahlmechanismus. Eine vorgefüllte Einweg-Fertigspritze umfasst den Produktbehälter und eine daran unlösbar befestigte Injektionsnadel und ist axial fixiert im Gehäuse des Autoinjektors gehalten. Der Autoinjektor, oder zumindest die Fertigspritze und der Spritzenhalter, sind entsprechend nur für einen einmaligen Gebrauch vorgesehen.

In einer bevorzugten Ausführungsform umfasst die Kupplung eine Kupplungshülse mit nach innen gerichteten Zähnen oder Vorsprüngen als ersten Kupplungselementen, welche radial in zweite Kupplungselemente eingreift, welche mit dem Antriebselement drehfest verbunden oder einstückig ausgebildet sind. Die zweiten Kupplungselemente umfassen nach aussen gerichtete Zähne oder Ausnehmungen am Antriebselement oder an einer mit dem Antriebselement und der Torsionsfeder drehfest verbundenen Federspule. Die Kupplungshülse ist eine eigenständige Komponente des Autoinjektors und umfasst insbesondere keine Verriegelungsglieder zur Verriegelung einer Nadelschutzhülse in einer Nadelschutzposition nach erfolgter Injektion.

In einer bevorzugten Ausführungsform weist die Kupplungshülse ein Halteelement auf welches durch eine Betätigungsbewegung oder einen Betätigungshub der Nadelschutzhülse freigegeben wird. Darauf wird die Kupplungshülse mit den ersten Kupplungselementen durch eine Feder, bevorzugt durch eine Nadelschutzfeder, in einem Kupplungshub axial und bevorzugt in proximaler Richtung relativ zum zweiten Kupplungselement bewegt und das Antriebselement freigegeben. Bevorzugt wird der Kupplungshub der Kupplungshülse durch einen nach distal gerichteten Anschlag am Mechanikhalter begrenzt, dadurch kann ein akustisches Signal erzeugt werden, welches dem Anwender den Beginn der Ausschüttung signalisiert.

In einer bevorzugten Ausführungsform ist ein Ende der Torsionsfeder rotationsfest an einen Schaft einer Federspule gekoppelt und begrenzt ein Federflansch einen Aufnahmebereich oder ein Volumen der Torsionsfeder distal. Die zweiten Kupplungselemente sind um mindestens die Länge oder Amplitude des Kupplungshubs vom distalen Federflansch beabstandet und an einer drehfest an das Antriebselement gekoppelten Erweiterung der Federspule umfassend eine Spulenhülse oder einen Spulenflansch angeordnet.

In einer bevorzugten Ausführungsform ist die Kupplungshülse eine Sperrhülse und weist ein Verriegelungsglied auf zur Verriegelung der Nadelschutzhülse in einer Nadelschutzposition am Ende der Injektion. Vorteilhafterweise sind die zweiten Kupplungselemente an einer Antriebshülse als Antriebselement angeordnet, und ihr proximales Ende ist um mindestens die Länge des Kupplungshubs von einem distalen Federflansch beabstandet. Die Antriebshülse ist mit einem Vortriebsglied in Form einer Kolbenstange in Gewindeeingriff.

Weiter bevorzugt verfügen Antriebshülse und Sperrhülse über eine Kulissensteuerung mit einem erste und zweiten Führungselement, beispielsweise einem radial ausgerichteten Führungsnocken welcher in eine Führungsnut eingreift. Die Führungselemente sind so ausgebildet und zusammenwirkend, dass nach Freigabe der Rotation der Antriebshülse eine initiale Drehung der Antriebshülse um höchstens 45° die Sperrhülse um einen Arretierhub von mindestens 1 mm in proximale Richtung drängt. Dadurch kann sichergestellt werden, dass auch bei ungünstigsten Toleranzen die Sperrhülse ihre Arretierposition sicher erreicht. Vorteilhafterweise ist der Führungsnocken an der Antriebshülse angebracht und die Führungsnut in der Sperrhülse vorgesehen und abgewinkelt zur Längsachse ausgerichtet.

Der Autoinjektor umfasst optional einen permanent eingebauten Rotationssensor zur kontinuierlichen Detektion von mindestens einer Rotationsposition, und bevorzugt von mindestens zwei Rotationspositionen pro Umdrehung des Antriebselementes während der Ausschüttung, sowie eine Prozessoreinheit zur Bestimmung einer axialen Kolbenposition des Kolbens im Produktbehälter oder eines Restvolumens im Produktbehälter aus den sukzessive detektierten Rotationspositionen. Es wird eine während der Ausschüttung durch die Torsionsfeder unmittelbar bewirkte Rotation eines Antriebsglieds kontinuierlich und bevorzugt mit einer Auflösung von einer halben Umdrehung oder weniger gemessen und daraus der Vortrieb und die Kolbenposition bestimmt. Durch eine verbesserte Auflösung kann das Restvolumen beziehungsweise die ausgeschüttete Menge an Medikament genau bestimmt werden, was insbesondere in Fällen von Bedeutung ist, in welchen die Ausschüttung nicht wie geplant verläuft oder gar durch den Anwender abgebrochen wird. Der Fortschritt einer Ausschüttung kann zudem in Echtzeit erfasst werden, und insbesondere Schwankungen in der Vortriebsgeschwindigkeit sind ohne Verzögerung erkennbar.

Der Autoinjektor umfasst weiter optional eine optische, akustische, oder taktile Signalisierungs- oder Anzeigeeinheit mit einem elektronisch angesteuerten Aktuator. Der Aktuator wird von der Prozessoreinheit nach Bestimmen einer axialen Kolbenposition, welche einer zumindest annähernd vollständigen Ausschüttung entspricht, und nach anschliessendem Ablauf einer vorbestimmten Haltezeit angesteuert oder aktiviert. Dadurch wird ein Signal erzeugt welches ein Ende der Injektion anzeigt und dem Anwender bestätigt, dass der Autoinjektor jetzt sicher von der Einstichstelle wegbewegt werden kann. Dementsprechend verfügt der Autoinjektor nicht über einen rein mechanisch ausgelösten Endklick, bei welchem ein Anschlagelement durch eine Feder beschleunigt wird. Die Haltezeit beträgt typischerweise einige wenige Sekunden, bevorzugt mindestens 3 Sekunden, und stellt sicher, dass die injizierte Menge an Medikament vollständig vom subkutanen Gewebe aufgenommen oder resorbiert wird und keine Flüssigkeit nach Wegbewegen des Autoinjektors durch die Einstichstelle auf die Gewebeoberfläche gelangt.

Der Autoinjektor weist bevorzugt ein Elektronikmodul auf mit einer Leiterplatine und darauf angeordnet einer Sensoreinheit zur Detektion von Zuständen oder Vorgängen, einer Prozessoreinheit zur Verarbeitung von Signalen der Sensoreinheit, einer Kommunikationseinheit zur drahtlosen Kommunikation von Daten der Prozessoreinheit an ein Drittgerät, und einer Batterie zur Speisung der vorgenannten Einheiten. Die Kommunikation mit einem stationären Drittgerät, beispielsweise einem Expertensystem in einer delokalisierten oder Cloud-basierten Infrastruktur, kann beispielsweise über ein 5G- oder 4G/LTE Mobilfunknetzwerk, insbesondere ein Narrowband Internet-of-Things NB-IoT erfolgen. Die Kommunikation mit einem mobilen Drittgerät, beispielsweise einem Mobiltelefon oder Smartphone, oder mit einem stationären Gateway zu einem drahtgebundenen Netzwerk, erfolgt bevorzugt über eine Bluetooth oder BLE Verbindung, welche vorteilhafterweise über ein Out-of-Band Pairing initiiert wird. Das Elektronikmodul umfasst weiter eine optische, akustische, und/oder taktile Anzeigeeinheit wie beispielsweise eine optische Anzeige, bei welcher ein Lichtleiter das Licht einer Lichtquelle auf der Leiterplatine an die Oberfläche des Gehäuses leitet. Ein durch die Anzeigeeinheit angezeigter Zustand kann Informationen zum Medikament, einen Gerätezustand des Autoinjektors, einen Modulzustand des Elektronikmoduls, oder einen Vorgangszustand eines laufenden oder abgeschlossenen Injektionsvorganges umfassen. Die Anzeigeeinheit des Elektronikmoduls kann einfach gehalten werden und sich auf einige wenige LEDs, beispielsweise in Ampelfarben oder zur Beleuchtung ausgewählter Piktogramme, und/oder einen akustischen Signalgenerator zur Erzeugung von sprachunabhängigen Geräuschen oder Melodien beschränken. Dies ist insbesondere im Zusammenwirken mit den fortgeschrittenen graphischen Anzeigemöglichkeiten und Sprachausgabemöglichkeiten eines Smartphones vorteilhaft, da das drahtlos an das Elektronikmodul gekoppelte Smartphone die verfeinerte, über eine Statusanzeige hinausgehende Kommunikation mit dem Anwender übernimmt.

### FIGUREN

In Zusammenhang mit den angehängten Figuren werden nachfolgend bevorzugte Ausführungsformen der Erfindung beschrieben. Diese sollen grundsätzliche Möglichkeiten der Erfindung aufzeigen und keinesfalls einschränkend ausgelegt werden. Es zeigen
- Fig.1: die Komponenten einer ersten Variante eines Autoinjektors,
- Fig.2: einen Längsschnitt durch den Autoinjektor aus Fig.1 vor der Injektion,
- Fig.3: einen Querschnitt durch den Autoinjektor aus Fig.2,
- Fig.4: einen Längsschnitt durch den Autoinjektor aus Fig.1 während der Injektion,
- Fig.5: einen Längsschnitt durch den Autoinjektor aus Fig.1 nach der Injektion,
- Fig.6: die Komponenten einer zweiten Variante eines Autoinjektors,
- Fig.7: einen Längsschnitt durch den Autoinjektor aus Fig.6 vor der Injektion,
- Fig.8: einen Querschnitt durch den Autoinjektor aus Fig.7,
- Fig.9: einen Längsschnitt durch den Autoinjektor aus Fig.6 während der Injektion,
- Fig.10: ein Antriebselement des Autoinjektors aus Fig.6, und
- Fig.11: einen Längsschnitt durch den Autoinjektor aus Fig.6 nach der Injektion.

### FIGURENBESCHREIBUNG

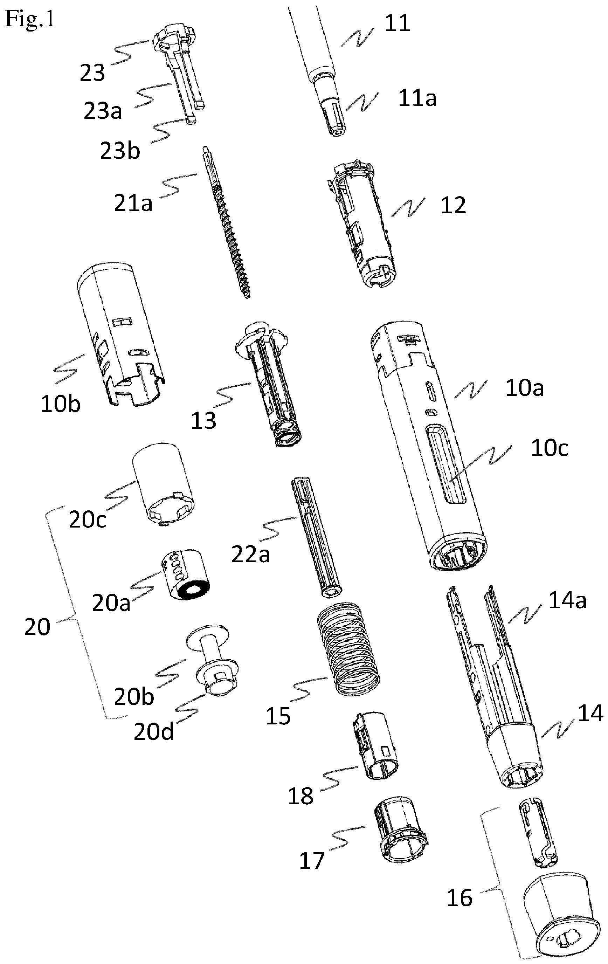

Fig.1 ist eine Explosionsdarstellung der Komponenten eines Autoinjektors nach einer ersten Variante der Erfindung. Der Autoinjektor weist ein hülsenförmiges, längliches Gehäuse mit einer Längsachse L und umfassend ein distales Gehäuseteil 10a und eine damit unlösbar verschnappte proximale Verschluss- oder Endkappe 10b. Das distale Gehäuseteil 10a und die Verschlusskappe 10b haben über die gesamte Länge des Autoinjektors denselben Querschnitt, und die Verschlusskappe umfasst eine einzige Endfläche senkrecht zur Längsachse. Ein Produktbehälter in Form einer Fertigspritze 11 mit einer am Produktbehälter unlösbar befestigten Injektionsnadel ist in einem Spritzenhalter 12 gehalten, wobei der Spritzenhalter im Gehäuse 10a axial- und drehfest aufgenommen ist. Die Fertigspritze 11 wird von einem Haltefederabschnitt eines fest im Gehäuse 10b verankerten Mechanikhalters 13 in distale Richtung in einen Eingriff mit einer Schulter des Spritzenhalters 12 gedrückt. Die Fertigspritze 11 ist in Bezug auf das Gehäuse 10a so angeordnet, dass die Spitze der Injektionsnadel um eine der subkutanen oder intramuskulären Einstechtiefe entsprechende Länge über das distale Ende des Gehäuses 10a hinausragt und durch eine Nadelschutzhülse 14 vor und nach der Injektion zumindest seitlich geschützt oder abgedeckt wird. Die Nadelschutzhülse 14 wird beim Einstechen der Injektionsnadel in die Injektionsstelle entlang der Längsachse L um einen Betätigungshub und gegen die Kraft einer Nadelschutzfeder 15 in proximale Richtung geschoben und löst dadurch eine Produktausschüttung aus wie weiter unten im Detail dargestellt. Die Nadelschutzhülse umfasst dazu zwei Hülsenarme 14a, welche gegenüber zwei als Sichtfenster bezeichneten Ausnehmungen 10c des Gehäuses um 90° um die Längsachse L versetzt beziehungsweise verdreht angeordnet sind. Nach der erfolgten Injektion kann die Nadelschutzhülse 14 relativ zu dem Gehäuse 10 aus der betätigten Position entlang der Längsachse L in die distale Richtung in eine Nadelschutzposition verschoben und dort gegen erneutes Zurückschieben blockiert werden. Die Nadelschutzfeder 15 ist eine als Druckfeder wirkende und als Wendelfeder ausgestaltete Feder aus Metall.

Ein Federpaket 20 umfasst eine Spiralfeder 20a, eine Federspule 20b, und eine Federhülse 20c. Die Spiralfeder 20a ist mit ihrem äusseren Ende drehfest an der Federhülse 20c verankert, welche wiederum drehfest im Gehäuse 10a aufgenommen ist. Das innere Ende der Spiralfeder 20a ist drehfest mit der Federspule 20b verbunden. Die Federspule 20b umfasst einen Federschaft und einen distalen und einen proximalen Federflansch, welche das Federvolumen axial begrenzen. Das Federpaket 20 kann als eigenständiges Bauteil vollständig vorgespannt im Gehäuse des Autoinjektors montiert werden und Spiralfedern unterschiedlicher Breite aufnehmen, wie in der eingangs genannten Patentanmeldung WO 2016/205963 ausführlich beschrieben. An dieser Stelle wird die WO 2016/205963 per Referenz vollständig in die vorliegende Anmeldung integriert.

Der Autoinjektor ist zur einfacheren Montage aus zwei Untereinheiten oder Baugruppen zusammensetzbar. In diesem Fall umfasst eine distale Spritzeneinheit des Autoinjektors ein erstes, distales Gehäuseteil 10a, die Nadelschutzhülse 14, die Gerätekappe 16 und den Spritzenhalter 12, während eine proximale Antriebseinheit die Verschlusskappe 10b, den Mechanikhalter 13, die Nadelschutzfeder 15, Schalthülse 17, Sperrhülse 18, Antriebs- und Vortriebselement, und das einmalig aufladbare Federpaket 20 für die automatische Substanzabgabe umfasst. In einem Abfüll- oder Montageprozess wird die Fertigspritze in die Spritzeneinheit eingeführt und die beiden Untereinheiten anschliessend zusammengeführt, wobei die beiden Gehäuseteile 10a, 10b unlösbar verschnappen. Das äussere Ende der Spiralfeder 20a ist an der Federhülse 20c oder, insbesondere falls keine Federhülse vorgesehen ist, am Mechanikhalter oder unmittelbar an der Verschlusskappe verankert. Ein Federflansch kann auch über seinen Aussenumfang an der Federhülse, am Mechanikhalter, oder am Gehäuse befestigt sein.

Die Fertigspritze 11 umfasst einen zylindrischen Spritzenkörper als Produktbehälter, an dessen distalen Ende eine hohle Injektionsnadel mit einer Spritzenschulter fest verbunden ist. Die Injektionsnadel der Fertigspritze ist von einer Nadelschutzkappe 11a abgedeckt, welche als sogenanntes Rigid Needle Shield (RNS) ausgebildet ist und ein gummielastisches Nadelschutzelement und eine Hülle aus Hartkunststoff umfasst. Die Nadelschutzkappe schützt die Injektionsnadel gegen mechanische Einwirkungen und Verschmutzung, und hält die Injektionsnadel und das Produkt steril. An dem distalen Ende des Autoinjektors ist in seinem Auslieferungszustand eine Geräte- oder Abziehkappe 16 angeordnet, die vor der Verwendung des Autoinjektors zusammen mit der Nadelschutzkappe 11a axial abgezogen und/oder abgedreht und vollständig entfernt wird. Der Spritzenhalter 12 umfasst zwei Finger, welche an ihren proximalen Enden an einer Halterhülse des Spritzenhalters befestigt sind und an ihren distalen Enden jeweils ein axiales Auflageelement für die Spritzenschulter aufweisen. Der gezeigte Spritzenhalter kann an den Durchmesser einer aufzunehmenden Fertigspritze mit einem nominalen Füllvolumen von 1.5 ml, 2.25 ml, oder 4 ml angepasst sein, so dass bei einem Wechsel der Spritzengrösse ausser dem Spritzenhalter keine Komponenten des Autoinjektors ausgetauscht werden müssen oder zumindest das distale Gehäuseteil 10a für alle Spritzengrössen dasselbe ist. Insbesondere für den kleinsten Spritzendurchmesser können die Finger flexibel sein und durch die Nadelschutzkappe bei axialem Einführen der Fertigspritze radial weggedrängt werden. Zur Aufnahme einer schmaleren Spritze kann der Spritzenhalter auch zweiteilig ausgeführt sein beziehungsweise durch einen Adapter ergänzt werden, wie dies in der Patentanmeldung PCT/EP2020/052127 offenbart ist. An dieser Stelle wird die PCT/EP2020/052127 per Referenz vollständig in die vorliegende Anmeldung integriert.

Die Spiralfeder 20a beziehungsweise die Federspule 20b versetzt ein Antriebselement 21 in eine Rotationsbewegung und ein Vortriebselement 22 in eine bevorzugt rein axiale Vortriebsbewegung. Dazu greift ein Gewindeelement in ein sich über den Ausschütthub erstreckendes Gewinde mit einer variablen Gewindesteigung. Das Gewindeelement umfasst ein Gewindesegment mit einer Ausdehnung in Dreh- oder Umfangrichtung von weniger als einer halben Windung, wobei vorzugsweise eine Flanke des Gewindesegments verschiedene Steigungswinkel aufweist, so dass jeweils ein anderer Bereich der Flanke des Gewindesegments vom Gewinde berührt wird wenn sich die Gewindesteigung mit fortschreitender Rotation ändert. Das variable Gewinde kann eine grössere Steigung im Anfangsbereich der Ausschüttung und eine kleinere Steigung am Ende aufweisen, so dass trotz abnehmender Federkraft eine konstante Ausschüttkraft resultiert, wie dies in der Patentanmeldung WO 2016/205961 offenbart ist. An dieser Stelle wird die WO 2016/205961 per Referenz vollständig in die vorliegende Anmeldung integriert.

Eine Schalthülse 17 ist formschlüssig mit einem proximalen Ende der Hülsenarme 14a der Nadelschutzhülse 14 und mit einem distalen Ende der Nadelschutzfeder 15 angeordnet und zumindest teilweise von Letzterer umgeben. Die Schalthülse 17 ist bevorzugt mit dem proximalen Ende der Hülsenarme der Nadelschutzhülse 14 verschnappt oder gar einstückig mit dieser ausgebildet. Innerhalb und koaxial zur Schalthülse 17 ist eine Sperrhülse 18 angeordnet, welche über ein sägezahnförmiges, an einem in distale Richtung weisenden Arm federnd angebrachtes Verriegelungsglied 18a derart an die Schalthülse 17 gekoppelt ist, dass eine Betätigungsbewegung von Nadelschutzhülse 14 und Schalthülse 17 auch die Sperrhülse 18 nach proximal bewegt. Durch einen zusätzlichen proximalen Arretierhub der Sperrhülse 18 relativ zur Schalthülse 17 in eine proximale Endposition wird das Verriegelungsglied 18a von der Schalthülse 17 für eine Bewegung nach innen sicher freigegeben. Durch die Federwirkung des Arms hintergreift das Verriegelungsglied 18a eine nach proximal gerichtete Kante des Autoinjektors oder rastet in eine axialfeste Ausnehmung des Autoinjektors ein und arretiert so die Sperrhülse 18 gegen eine distale Bewegung. Beim Entfernen des Autoinjektors von der Einstichstelle wird die Schalthülse 17 durch die Nadelschutzfeder 15 in distaler Richtung über das Verriegelungsglied 18a geschoben, worauf das Verriegelungsglied durch die Federwirkung des Arms in einer Verriegelungsposition eine nach proximal gerichtete Kante der Schalthülse 17 hintergreift und die Schalthülse sowie die Nadelschutzhülse gegen eine erneute Bewegung in proximale Richtung verriegelt oder blockiert.

Der Spritzenhalter 12 ist bevorzugt aus einem transparenten Material gefertigt so dass der Inhalt der Spritze durch die Sichtfenster 10c im Gehäuse 10a optisch inspiziert werden kann. Eine im Bereich des Sichtfensters bevorzugt durch eine variable Dicke im Material des Spritzenhalters gebildete Lupe erlaubt es Schwebeteilchen in der Flüssigkeit zu identifizieren und den Medikamentenzustand zu beurteilen. Zum Schutz der Flüssigkeit vor Sonnenlicht kann eine überlange, über den Bereich der Sichtfenster bis in die proximale Hälfte des Autoinjektors reichende Gerätekappe vorgesehen sein.

In der ersten Variante nach Fig.1 umfasst der Autoinjektor ein Antriebselement oder ein Rotationsglied in Form einer Gewindestange 21a mit einem Aussengewinde, welches sich zumindest über eine dem Ausschütthub entsprechende Länge erstreckt. Die Gewindestange 21a ist drehfest mit dem Federschaft 20b gekoppelt oder sogar einstückig mit diesem ausgebildet. Eine Vortriebselement in Form einer Vortriebshülse 22a weist an einem proximalen Ende auf einer Innenseite ein Gewindeelement auf zum Eingriff in das Aussengewinde, umfassend einen Gewindeabschnitt mit bevorzugt weniger Windungen als das Aussengewinde, oder ein Gewindesegment mit einer Ausdehnung in Drehrichtung weniger als einer Windung, bevorzugt weniger als einer halben Windung. Durch eine axiale Nut oder eine sonstige Abweichung von einer rotationssymmetrischen Aussenseite ist die Vortriebshülse 22a im Mechanikhalter 13 oder im Gehäuse gegen eine Rotation gesichert, so dass die durch die Rotationsfeder 20a erzeugte Rotation des Antriebselements in eine lineare Vortriebsbewegung umgesetzt wird. Alternativ kann die Vortriebshülse 22a ein Innengewinde aufweisen, welches sich über eine dem Ausschütthub entsprechende Länge erstreckt, und das Gewinde der Gewindestange 21a reduziert sein auf ein Gewindeelement zum Eingriff in das Innengewinde, umfassend einen Gewindeabschnitt mit bevorzugt weniger Windungen als das Innengewinde, oder ein Gewindesegment mit einer Länge von weniger als einer Windung, bevorzugt weniger als einer halben Windung. Eine Kupplungshülse 23 weist einen hohlzylindrischen Körper auf und ein Halteelement mit zwei in distaler Richtung angebrachten flexiblen Haltearmen 23a, an deren Ende sich je ein radial nach innen stehender Haltenocken 23b befindet. Die Federspule 20b weist eine distale Erweiterung 20d auf umfassend eine konzentrisch zum Federschaft angeordnete Spulenhülse mit gegen Rotation um die Längsachse, tangential wirkenden und radial nach aussen gerichteten Sperrflächen an vier über den Umfang der Spulenhülse verteilten Ausprägungen. Die Sperrflächen können auch von Einprägungen an der Peripherie eines konzentrisch zum Federschaft angeordneten Spulenflanschs der Erweiterung 20d gebildet werden.

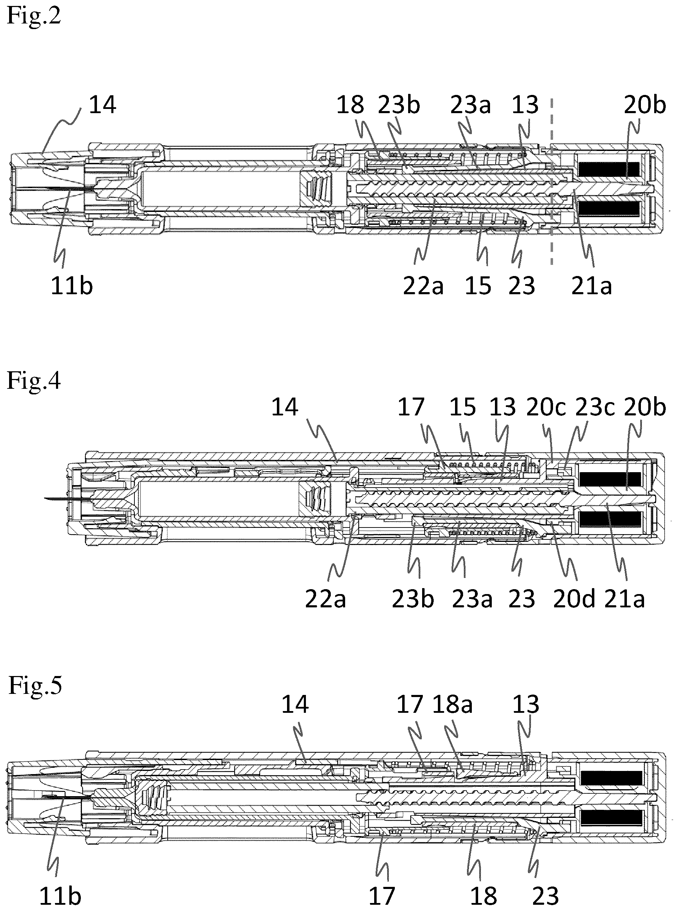

Fig.2 zeigt einen Längsschnitt durch den Autoinjektor nach Fig.1 im injektionsbereiten Zustand, nach Abzug der Gerätekappe, in welchem die Injektionsnadel 11b seitlich von der Nadelschutzhülse 14 abgedeckt ist. Vor der Ausschüttung greifen die Haltenocken 23b des Halteelements in Ausnehmungen des axialfesten Mechanikhalters 13 ein und sind durch einen Innenumfang der Sperrhülse 18 an einer Bewegung nach aussen gehindert, wodurch sich die Kupplungshülse 23 auch axial nicht bewegen kann. Beim Auslösen der Ausschüttung wird die Sperrhülse 18 durch eine proximale Bewegung der Nadelschutzhülse 14 von der Position der Ausnehmungen wegbewegt, so dass sich die Haltearme 23a radial lösen können und die Kupplungshülse 23 freigeben.

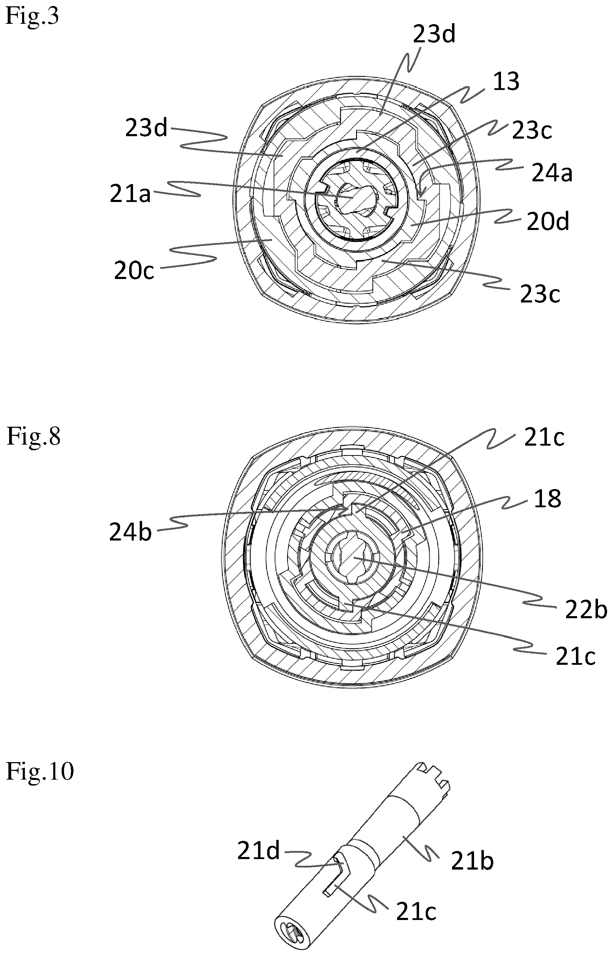

Fig.3 zeigt einen Querschnitt durch den Autoinjektor axial auf Höhe eines proximalen Endes der Kupplungshülse vor der Ausschüttung, in Fig.2 durch eine unterbrochene senkrechte Linie angedeutet. Die Kupplungshülse umfasst an dieser Stelle vier jeweils um 90° versetzte und nach innen gerichtete Vorsprünge 23c als erstes Kupplungselement, welche über radial ausgerichtete Sperrflächen 24a in vier korrespondierende Ausnehmungen an einer distalen Erweiterung 20d der Federspule als zweites Kupplungselement rotationsfest eingreifen. Gleichzeitig greifen vier gleichmässig über den Umfang verteilte und nach aussen gerichtete Ausprägungen 23d der Kupplungshülse in vier Ausnehmungen einer rotationsfest im Gehäuse montierten Federhülse 20c. Die Vorsprünge 23c und Ausprägungen 23d haben eine Winkelausdehnung von ungefähr 45°, zudem sind die nach innen gerichteten Vorsprünge gegen die nach aussen gerichteten Ausprägungen versetzt, so dass sich eine mehr oder weniger konstante Dicke der Kupplungshülse ergibt. Wie aus Fig.3 ersichtlich könnte an Stelle der abwechslungsweise innen und aussen angeordneten Vorsprünge/Ausprägungen der Kupplungshülse auch von wechselseitig angeordneten Ausnehmungen/Einprägungen der Kupplungshülse gesprochen werden. Durch die beiden Eingriffe ist die Federspule 20b rotationsfest mit dem Gehäuse 10b gekoppelt, somit bewegt sich auch weder die Gewindestange 21a noch die Vortriebshülse 22a.

Fig.4 zeigt einen kombinierten Längsschnitt durch den Autoinjektor unmittelbar nach Auslösung beziehungsweise zu Beginn der Ausschüttung. Die Schnittebene der unteren Hälfte entspricht der Schnittebene aus Fig.2 und ist gegenüber der Schnittebene der oberen Hälfte um 60° um die Längsachse gedreht. Die Nadelschutzhülse 14 ist durch Kontakt mit der Injektionsstelle nach proximal verschoben und drängt dadurch auch die Sperrhülse 18 in proximale Richtung, unter Spannung der Nadelschutzfeder 15. Die Haltearme 23a der Kupplungshülse 23 werden dadurch für eine erste Lösebewegung nach radial aussen freigegeben, und die Kupplungshülse kann sich axial bewegen. Das proximale Ende der Nadelschutzfeder 15 stützt sich an der Kupplungshülse 23 ab und schiebt diese um einen Kupplungshub nach proximal, wobei der Kupplungshub in dieser Variante vom Betätigungshub der Nadelschutzhülse 14 verschieden ist. Bevorzugt wird dabei über radial nach aussen gerichtete Vorsprünge an den Haltearmen 23a die Sperrhülse 18 gegenüber der Schalthülse 17 um einen Arretierhub entsprechend dem Kupplungshub bis zu einem ersten Anschlag mit dem Mechanikhalter 13 nach proximal bewegt. In dieser Endposition der Sperrhülse wird die Verriegelung der Nadelschutzhülse aktiviert, indem das Verriegelungsglied 18a mit einem nach innen gerichteten Vorsprung in eine Ausnehmung des Mechanikhalters einrastet und eine distale Bewegung des Sperrelements verunmöglicht. Falls der erste Anschlag nicht durch die Kupplungshülse 23 sondern durch die Sperrhülse 18 erfolgt, steht die Kupplungshülse am Ende der Ausschüttung für einen finalen Hub bis zu einem zweiten Anschlag und damit für einen mechanischen Endklick zur Verfügung. Dazu werden die Haltearme 23a von einer proximalen Endkante der Vortriebshülse 22a für eine zweite Lösebewegung nach radial innen freigegeben. Da die Sperrflächen der inneren Vorsprünge 23c der Kupplungshülse und der Ausnehmungen der Erweiterung 20d der Federspule je eine axiale Ausdehnung oder eine Überlappung von weniger als dem Kupplungshub aufweisen werden dadurch die Eingriffe der Vorsprünge der Kupplungshülse mit den Ausnehmungen der Erweiterung gelöst, und die Federspule beginnt unter Einwirkung der Torsionsfeder zu rotieren. Die Sperrflächen der Erweiterung 20d der Federspule befinden sich an Einprägungen eines Spulenflansch welcher in distaler Richtung um mindestens den Kopplungshub vom distalen Federflansch abgesetzt ist, oder an Ausprägungen auf einer Spulenhülse welche in distaler Richtung um mindestens den Kopplungshub vom distalen Federflansch abgesetzt sind.

Fig.5 zeigt einen kombinierten Längsschnitt durch den Autoinjektor nach der Injektion. Die Schnittebene der unteren Hälfte entspricht der Schnittebene aus Fig.2 und ist gegenüber der Schnittebene der oberen Hälfte um 90° um die Längsachse gedreht. Die Nadelschutzhülse 14, welche in einer Sicherungsbewegung beim Entfernen des Autoinjektors von der Einstichstelle aus einer hinteren Endposition durch eine Nadelschutzfeder in eine vordere Endposition bewegt wird, deckt die Injektionsnadel 11b seitlich ab. Nach radial innen gerichtete Vorsprünge an federnden Armen des Verriegelungsglieds 18a greifen in Ausnehmungen des Mechanikhalters 13, wodurch eine Bewegung der Sperrhülse 18 in zumindest distaler Richtung verhindert wird. Die Sperrhülse steht proximal an einer distalen Stirnfläche des Mechanikhalters an, so dass sich die Sperrhülse auch nicht in proximaler Richtung bewegen kann. Nach radial aussen gerichtete Vorsprünge an den federnden Armen des Verriegelungsglieds 18a hintergreifen eine proximale Kante der Schalthülse 17, so dass die Schalthülse ebenfalls nicht in proximale Richtung bewegt werden kann. Die axialfeste Arretierung der Sperrhülse kann auch durch ein von der Verriegelungshülse verschiedenes Arretier- oder Schnappelement erfolgen.

Die inneren und die äusseren Prägungen der Kupplungshülse und ihrer jeweiligen Gegenstücke können unterschiedlich sein in Ausbildung, Anzahl und/oder axialer Anordnung. Beispielsweise können die Ausprägungen die Form von axialen Rippen annehmen und die Ausnehmungen an Federspule oder -hülse entsprechend die Form von axialen Schlitzen, oder sowohl Ausprägungen als auch Ausnehmungen sind als Zähne gestaltet. Die Ausnehmungen an der Federhülse können auch unmittelbar am Gehäuse angebracht sein, die entsprechende Verbindung kann, muss aber nicht beim Kopplungshub ebenfalls gelöst werden. In Anbetracht des einmaligen Einsatzes des Autoinjektors und der Rotationsblockierung können die inneren und äusseren Ausprägungen der Kupplungshülse auch jeweils unter sich unterschiedlich ausgestaltet sein, so lange nur die axiale Ausdehnung und Anordnung der inneren Vorsprünge ein Lösen des Eingriffs durch einen Kupplungshub erlaubt und die äusseren Ausprägungen mit der rotativen Ausrichtung der Haltearme der Kupplungshülse kompatibel sind.

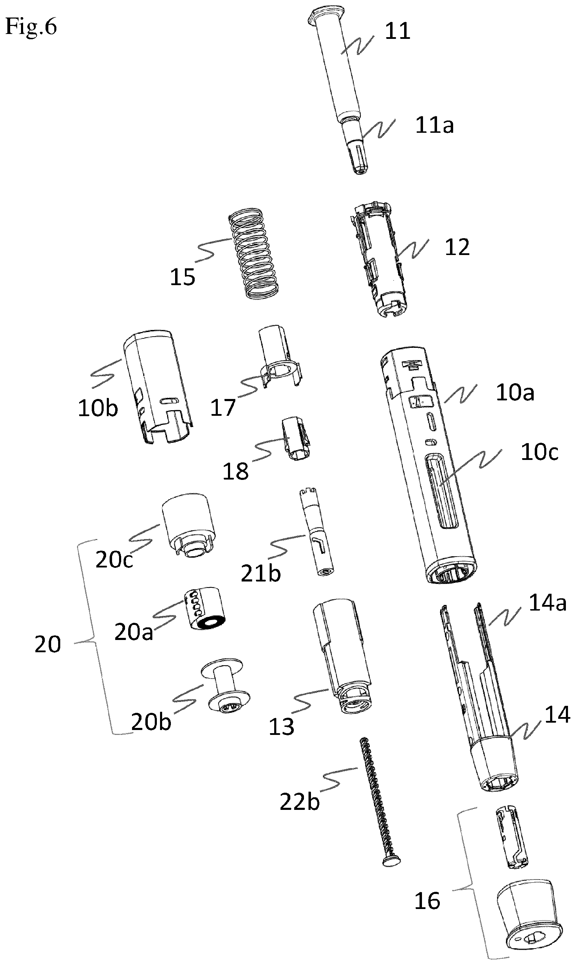

Fig.6 ist eine Explosionsdarstellung der Komponenten eines Autoinjektors nach einer zweiten Variante der Erfindung. Die im Vergleich zu Fig.1 unterschiedlichen Komponenten umfassen ein Antriebselement in Form einer Antriebshülse 21b welche distal der Torsionsfeder 20a dreh- und axialfest mit der Federspule 20b verbunden ist oder gar einstückig mit dieser ausgebildet ist. Die Antriebshülse weist an ihrem distalen Ende ein Gewindeelement auf welches in ein sich über eine dem Ausschütthub entsprechende Länge erstreckendes Aussengewinde einer Kolbenstange 22b eingreift. Das Gewindeelement umfasst einen Gewindeabschnitt eines Innengewindes mit bevorzugt weniger Windungen als das Aussengewinde, oder ein Gewindesegment mit einer Ausdehnung in Drehrichtung von weniger als einer Windung, bevorzugt weniger als einer halben Windung. Die Kolbenstange befindet sich in einem Innenraum der Antriebshülse und fungiert als Vortriebselement, sie weist eine Längsnut auf, in welche der gehäusefeste Mechanikhalter 13 eingreift, so dass durch eine Rotationsbewegung der Antriebshülse in bekannter Weise eine rein axiale Vorwärtsbewegung der Kolbenstange resultiert. Alternativ kann das hülsenförmige Antriebselement ein Innengewinde aufweisen, welches sich über eine dem Ausschütthub entsprechende Länge erstreckt, und das Gewinde der Gewindestange am proximalen Ende auf einen Gewindeabschnitt mit wenigen Windungen reduziert sein oder einen Gewindesegment von weniger als einer Windung umfassen.

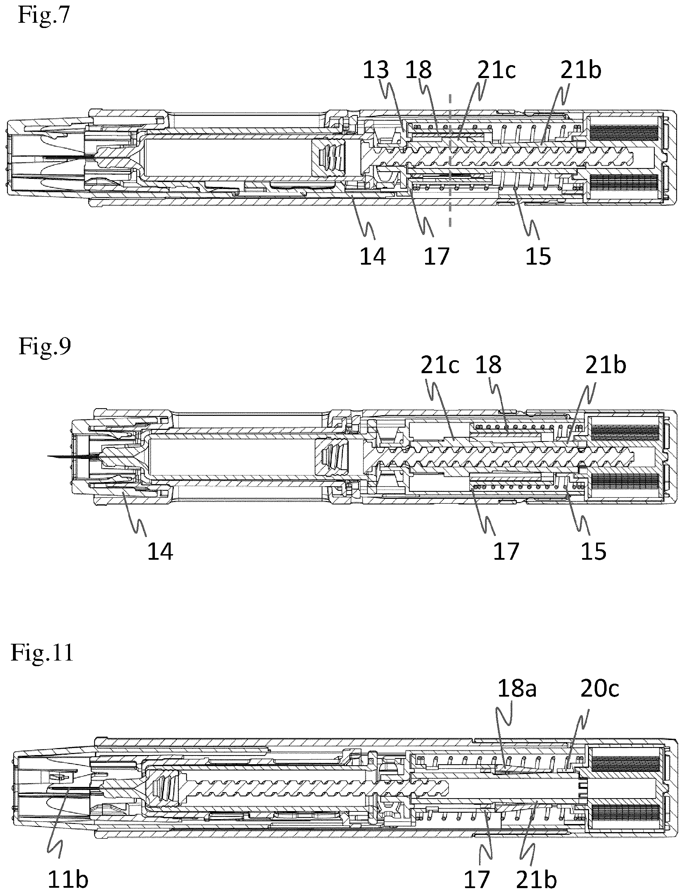

Fig.7 zeigt einen kombinierten Längsschnitt durch den Autoinjektor nach Fig.6 im injektionsbereiten Zustand, nach Abzug der Gerätekappe. Die Schnittebene der unteren Hälfte ist gegenüber der Schnittebene der oberen Hälfte um 90° um die Längsachse gedreht. Die Schalthülse 17, welche von der Nadelschutzfeder 15 in distaler Richtung an ein proximales Ende der Nadelschutzhülse 14 gedrängt wird, ist im Mechanikhalter 13 drehfest geführt. Die Sperrhülse 18 ist rotationsfest an die Schalthülse 17 gekoppelt. Die Antriebshülse 21b weist an ihrer Aussenseite als zweites Kupplungselement eine Ausprägung auf, in Form von zwei gegenüberliegenden axial ausgerichteten Sperrrippen 21c.

Fig.8 zeigt in Blickrichtung distal einen Querschnitt durch den Autoinjektor axial auf Höhe der Ausprägungen der Antriebshülse, in Fig.7 durch eine unterbrochene senkrechte Linie angedeutet. Die Sperrhülse 18 umfasst an dieser Stelle zwei Sperrnuten, in welche die Sperrrippen 21c über Sperrflächen 24b eingreifen und welche eine Rotation der Antriebshülse zumindest in Entspannungsrichtung der Torsionsfeder (in Blickrichtung der Fig.8 im Gegenuhrzeigersinn) und damit einen Vortrieb der Kolbenstange 22b verhindern oder blockieren. Durch eine axiale Bewegung der Sperrhülse 18 beim Einstechen um einen Kupplungshub, welcher in dieser Variante dem Betätigungshub entspricht, wird der Eingriff gelöst und die Antriebshülse zur Rotation freigegeben.

Alternativ zum dargestellten Eingriff mittels zwei Sperrrippen / Sperrnuten können auch andere Ausprägungen oder Vorsprünge an der Antriebshülse als zweites Kupplungselement dienen, und in korrespondierende Ausnehmungen oder Einprägungen der Sperrhülse als erstem Kupplungselement rotationsfest eingreifen, immer vorausgesetzt, dass die axiale Anordnung und Ausdehnung der Sperrflächen beim Kupplungshub der Sperrhülse getrennt werden.

Fig.9 zeigt einen Längsschnitt durch den Autoinjektor unmittelbar nach Auslösung beziehungsweise zu Beginn der Ausschüttung. Die Nadelschutzhülse 14 ist durch Kontakt mit der Injektionsstelle nach proximal verschoben und drängt dadurch auch die Schalthülse 17 und die Sperrhülse 18 in proximale Richtung, unter Spannung der Nadelschutzfeder 15 und unter Freigabe der Antriebshülse 21b wie beschrieben.

Die Antriebshülse 21b kann über eine weitere Ausprägung in Form eines Zapfens oder Nockens verfügen, welche als erstes Führungselement nach erfolgtem Kupplungshub in ein zweites Führungselement in Form einer Führungsnut der Sperrhülse 18 eingreift. Diese Führungselemente bewirken als Kulissensteuerung, dass eine initiale Drehung der Antriebshülse die Sperrhülse 18 gegenüber der Schalthülse 17 um einen Arretierhub weiter nach proximal bewegt wird in eine Endposition der Sperrhülse, in welcher die Verriegelung der Nadelschutzhülse aktiviert wird indem das Verriegelungsglied 18a mit einem nach innen gerichteten Vorsprung in eine umlaufende Nut der axialfesten Antriebshülse 21b einrastet. Die dabei eingenommene axiale Endposition der Sperrhülse ist in Fig. 9 erkennbar, in dieser Position ist die Sperrhülse axial arretiert. Die Führungsnut ist nicht parallel zur Längsachse des Autoinjektors orientiert beziehungsweise diesbezüglich abgewinkelt oder in Form eines Schraubenlinienabschnitts ausgebildet.

Fig.10 zeigt eine Variante des ersten Führungselements in Form einer Verlängerung 21d in proximale Richtung der Ausprägung welche die Sperrrippe 21c bildet. Der Bereich der Verlängerung ist gegenüber der Längsachse abgewinkelt, dementsprechend ist die in Fig.8 dargestellte Sperrnut der Sperrhülse so breit ausgebildet, dass sie als zweites Führungselement die Verlängerung 21d ebenfalls aufnehmen kann. Sobald gegen Ende des Kupplungshubs die axiale Sperrrippe 21c axial aus der Sperrnut austritt und die Sperrflächen entkoppelt, beginnen die Antriebshülse 21b und damit eine gegenüber der Längsachse abgewinkelte Fläche der Verlängerung 21d zu rotieren. Die Sperrhülse wird dadurch weiter in proximale Richtung geschoben, insbesondere gegenüber der Schalthülse, bis das Verriegelungsglied der Sperrhülse hinter einer umlaufenden Kante der Antriebshülse einrastet und eine Bewegung der Sperrhülse in distale Richtung verunmöglicht. Ein abgewinkeltes erstes Führungselement muss nicht unmittelbar an die Sperrrippe 21c anschliessen sondern kann auch in Umfangsrichtung versetzt sein und sich in einer separaten, von der Sperrnut verschiedenen Führungsnut bewegen.

Fig.11 zeigt einen Längsschnitt durch den Autoinjektor nach der Injektion. Die Nadelschutzhülse 14, welche in einer Sicherungsbewegung beim Entfernen des Autoinjektors von der Einstichstelle aus einer hinteren Endposition durch eine Nadelschutzfeder in eine vordere Endposition bewegt wird, deckt die Injektionsnadel 11b seitlich ab. Das Verriegelungsglied 18a mit nach radial innen gerichteten Vorsprüngen an federnden Armen der Sperrhülse greift in eine umlaufende Ausnehmung der Antriebshülse 21b, wodurch eine Bewegung der Sperrhülse in zumindest distaler Richtung verhindert wird. Die Sperrhülse steht proximal an einer distalen Stirnfläche der Federhülse 20c an, so dass sich die Sperrhülse auch nicht in proximaler Richtung bewegen kann. Radial gerichtete Vorsprünge an den federnden Armen hintergreifen eine proximale Kante der Schalthülse 17, so dass die Schalthülse ebenfalls nicht in proximale Richtung bewegt werden kann.

**BEZUGSZEICHENLISTE**

| | | | |
|---|---|---|---|
| 10a | Gehäuseteil | 20b | Federspule |
| 10b | Verschlusskappe | 20c | Federhülse |
| 10c | Ausnehmung | 20d | Erweiterung |
| 11 | Fertig spritze | 21 | Antriebselement |
| 11a | Nadelschutzkappe | 21a | Gewindestange |
| 11b | Injektionsnadel | 21b | Antriebshülse |
| 12 | Spritzenhalter | 21c | Ausprägung |
| 13 | Mechanikhalter | 21d | Führungselement |
| 14 | Nadelschutzhülse | 22 | Vortriebselement |
| 14a | Arm | 22a | Vortriebshülse |
| 15 | Nadelschutzfeder | 22b | Kolbenstange |
| 16 | Gerätekappe | 23 | Kupplungshülse |
| 17 | Schalthülse | 23a | Haltearm |
| 18 | Sperrhülse | 23b | Haltenocken |
| 18a | Verriegelungsglied | 23c | Vorsprung |
| 20 | Federpaket | 23d | Ausprägung |
| 20a | Spiralfeder | 24 | Sperrfläche |

## Patentansprüche

1. Autoinjektor mit einem Gehäuse (10a, 10b), einem im Gehäuse axial fixierten Produktbehälter (11), einer Torsionsfeder (20a), einem Antriebselement (21, 21a, 21b), einem Vortriebselement (22, 22a, 22b), und einer Nadelschutzhülse (14), wobei bei Anpressen des Autoinjektors an eine Injektionsstelle die Nadelschutzhülse (14) eine Betätigungsbewegung in proximale Richtung ausführt, und wobei zur Ausschüttung von Flüssigkeit aus dem Produktbehälter (11) durch eine Injektionsnadel (11b) in die Injektionsstelle die Torsionsfeder (20a) das Antriebselement (21) in Rotation versetzt und das rotierende Antriebselement eine Bewegung des Vortriebselements (22) und eines Kolbens im Produktbehälter in distaler Richtung bewirkt,
**dadurch gekennzeichnet, dass** der Autoinjektor eine Kupplung umfasst, welche als Folge der Betätigungsbewegung der Nadelschutzhülse (14) das Antriebselement (21) zur Rotation freigibt.

2. Autoinjektor nach Anspruch 1, **dadurch gekennzeichnet dass** die Kupplung ein erstes Kupplungselement (23c) umfasst, welches über eine Sperrfläche (24a, 24b) in ein zweites Kupplungselement (21c) eingreift, wobei dieser Eingriff durch einen axialen Kupplungshub der beiden Kupplungselemente lösbar ist, bevorzugt durch einen axialen Kupplungshub entsprechend mindestens einer Einstechtiefe der Injektionsnadel (11b).

3. Autoinjektor nach Anspruch 2, **dadurch gekennzeichnet, dass** das Vortriebselement (22) ein Axialführungselement aufweist für eine ausschliesslich lineare Vortriebsbewegung im Gehäuse (10a).

4. Autoinjektor nach Anspruch 3, **dadurch gekennzeichnet, dass** das Antriebselement eine Gewindestange (21a) und das Vortriebselement eine Vortriebshülse (22a) umfasst.

5. Autoinjektor nach einem der Ansprüche 2 bis 4, **dadurch gekennzeichnet dass** die Kupplung eine Kupplungshülse (23) mit dem ersten Kupplungselement (23c) umfasst, welches radial in ein mit dem Antriebselement (21) drehfest verbundenes zweites Kupplungselement eingreift.

6. Autoinjektor nach Anspruch 5, **dadurch gekennzeichnet dass** die Kupplungshülse (23) ein Halteelement (23a, 23b) aufweist, welches durch die Betätigungsbewegung der Nadelschutzhülse (14) freigegeben wird, und dass der Autoinjektor eine Feder (15) aufweist, welche die Kupplungshülse (23) anschliessend in einem Kupplungshub axial relativ zum zweiten Kupplungselement bewegt.

7. Autoinjektor nach Anspruch 5, wobei ein Ende der Torsionsfeder (20a) rotationsfest an eine Federspule (20b) gekoppelt ist und ein Federflansch einen Aufnahmebereich der Torsionsfeder distal begrenzt, **dadurch gekennzeichnet, dass** das zweite Kupplungselement an einer Erweiterung (20d) der Federspule (20b) angeordnet und um mindestens die Länge des Kupplungshubs vom distalen Federflansch beabstandet ist.

8. Autoinjektor nach Anspruch 2 oder 3, umfassend eine Sperrhülse (18) mit einem Verriegelungsglied (18a) zur Verriegelung der Nadelschutzhülse (14) in einer Nadelschutzposition am Ende der Injektion, und **dadurch gekennzeichnet dass** das erste Kupplungselement an der Sperrhülse (18) angeordnet ist.

9. Autoinjektor nach Anspruch 8, **dadurch gekennzeichnet dass** das Antriebselement eine Antriebshülse (21b) und das Vortriebselement eine Kolbenstange (22b) umfasst, und dass das zweite Kupplungselement (21c) an der Antriebshülse (21b) angeordnet ist.

10. Autoinjektor nach Anspruch 9, **dadurch gekennzeichnet dass** die Antriebshülse (21b) und die Sperrhülse (18) eine Kulissensteuerung mit einem ersten und zweiten Führungselement aufweisen, welche so ausgebildet ist, dass eine initiale Drehung der Antriebshülse (21b) die Sperrhülse (18) um einen Arretierhub in proximale Richtung drängt.

11. Autoinjektor nach Anspruch 2, **dadurch gekennzeichnet dass** das erste und das zweite Kupplungselement mindestens zwei Sperrflächen (24) an Ausprägungen beziehungsweise korrespondierenden Ausnehmungen umfassen.

12. Autoinjektor nach einem der vorhergehenden Ansprüche, umfassend einen Rotationssensor zur Detektion von mindestens einer Rotationsposition pro Umdrehung des Antriebselementes während der Ausschüttung, sowie eine Prozessoreinheit zur Bestimmung einer axialen Kolbenposition des Kolbens im Produktbehälter aus den detektierten Rotationspositionen.

13. Autoinjektor nach Anspruch 12, umfassend eine Kommunikationseinheit zur Kommunikation mit einem Drittgerät und/oder eine Anzeigeeinheit zur Anzeige eines Zustandes des Autoinjektors.

14. Antriebseinheit für einen Autoinjektor, umfassend eine Verschlusskappe (10b), eine Torsionsfeder (20a) zur einmaligen Ausschüttung eines maximalen Inhalts eines im Autoinjektor axial fixierten Produktbehälters, ein Antriebselement (21, 21a, 21b), und ein Vortriebselement (22, 22a, 22b), wobei zur Ausschüttung von Flüssigkeit aus dem Produktbehälter die Torsionsfeder (20a) das Antriebselement (21) in Rotation versetzen und das rotierende Antriebselement eine Bewegung des Vortriebselements (22) und eines Kolbens im Produktbehälter in distaler Richtung bewirken kann,
**dadurch gekennzeichnet, dass** die Antriebseinheit eine Kupplung umfasst mit einem ersten Kupplungselement (23c), welches über eine Sperrfläche (24a, 24b) in ein zweites Kupplungselement (21c) eingreift, wobei dieser Eingriff durch einen axialen Kupplungshub der beiden Kupplungselemente lösbar ist und das Antriebselement (21) zur Rotation freigeben kann.

15. Antriebseinheit nach Anspruch 14, **dadurch gekennzeichnet dass** der Kupplungshub durch eine Betätigungsbewegung einer Nadelschutzhülse des Autoinjektors erfolgt und mindestens einer Einstechtiefe einer Injektionsnadel entspricht.
